# EUROPEAN PATENT APPLICATION

(11) **EP 2 479 185 A1**
(43) Date of publication of application: **25.07.2012**
(21) Application number: 11305058.7
(22) Date of filing: 20.01.2011
(51) Int. Cl.: C07K 14/01, B82Y 5/00

(54) **Homomultimeric structure by assembly of SIRV2 P98 proteins or P98 variants, conjugate and uses thereof**

(71) Applicant: INSTITUT PASTEUR, 75724 Paris Cedex 15 (FR)
(72) Inventor: Quax, Tessa E.F., 75015, Paris (FR); Lucas, Soizick, 92320, Chatillon (FR); Forterre, Patrick, 92340, Bourg La Reine (FR); Prangishvili, David, 78000, Versailles (FR)
(74) Representative: Desaix, Anne

(57) **Abstract**

The present invention relates to a homomultimeric protein structure constituted by assembled monomers of the P98 protein of *Sulfolobus islandicus* rod-shaped virus 2 (SIRV2) or assembled monomer variants of said P98 protein. In a particular embodiment, this homomultimeric protein structure has a seven-fold rotational symmetry, and is found in an open conformation or closed conformation. A particular structure has the form of baseless 7-face pyramid. The invention also relates to a conjugate comprising or consisting of a homomultimeric protein structure of the invention to which one or more heterologous molecule(s) is attached. Furthermore, the invention also concerns a homomultimeric protein structure or a conjugate of the invention, inserted into or exposed at the surface of a lipid layer or bilayer, of a vesicle or of a cell, and their uses thereof.

SEQUENCE LISTING

<110>  INSTITUT PASTEUR

<120>  HOMOMULTIMERIC STRUCTURE BY ASSEMBLY OF SIRV2 P98  PROTEINS OR
       P98 VARIANTS, CONJUGATE AND USES THEREOF

<130>  B09304 - AD/LV/KN

<140>  EP XXXXXX
<141>  2011-01-20

<160>  7

<170>  PatentIn version 3.3

<210>  1
<211>  297
<212>  DNA
<213>  SIRV2

<220>
<221>  CDS
<222>  (1)..(297)
<223>  P98 protein

<400>  1
atg gct ata aca tta tta gaa gga gca cta tat gga ttt ttt gca gta        48
Met Ala Ile Thr Leu Leu Glu Gly Ala Leu Tyr Gly Phe Phe Ala Val
1               5                   10                  15

aca gga ata tta gtg ggc agt ttt gta gtt gga gaa att gtc cac tta        96
Thr Gly Ile Leu Val Gly Ser Phe Val Val Gly Glu Ile Val His Leu
            20                  25                  30

tat aat gaa aaa caa aat aat gag aat ttt gct aag gct gtt gat caa       144
Tyr Asn Glu Lys Gln Asn Asn Glu Asn Phe Ala Lys Ala Val Asp Gln
        35                  40                  45

atg tca aag agt aca gtt att gct gtt gaa agt atc aaa gat act act       192
Met Ser Lys Ser Thr Val Ile Ala Val Glu Ser Ile Lys Asp Thr Thr
    50                  55                  60

aca act gct att aat gca tta ctc aat atg gat act tta aga gat gta       240
Thr Thr Ala Ile Asn Ala Leu Leu Asn Met Asp Thr Leu Arg Asp Val
65                  70                  75                  80

aat gcg tta gct aga gaa aaa gca aaa gat caa aat cca aat acg caa       288
Asn Ala Leu Ala Arg Glu Lys Ala Lys Asp Gln Asn Pro Asn Thr Gln
                85                  90                  95

gct aaa taa                                                           297
Ala Lys

<210>  2
<211>  98
<212>  PRT
<213>  SIRV2

<400>  2

Met Ala Ile Thr Leu Leu Glu Gly Ala Leu Tyr Gly Phe Phe Ala Val
1               5                   10                  15

Thr Gly Ile Leu Val Gly Ser Phe Val Val Gly Glu Ile Val His Leu

20                  25                  30

Tyr Asn Glu Lys Gln Asn Asn Glu Asn Phe Ala Lys Ala Val Asp Gln
        35                  40                  45

Met Ser Lys Ser Thr Val Ile Ala Val Glu Ser Ile Lys Asp Thr Thr
    50                  55                  60

Thr Thr Ala Ile Asn Ala Leu Leu Asn Met Asp Thr Leu Arg Asp Val
65                  70                  75                  80

Asn Ala Leu Ala Arg Glu Lys Ala Lys Asp Gln Asn Pro Asn Thr Gln
                85                  90                  95

Ala Lys

<210>  3
<211>  98
<212>  PRT
<213>  Artificial

<220>
<223>  P98 variant

<400>  3

Met Ala Ile Thr Leu Leu Glu Gly Ala Leu Tyr Gly Phe Phe Ala Val
1               5                   10                  15

Thr Gly Ile Leu Ile Gly Ser Phe Val Val Gly Glu Ile Val His Leu
            20                  25                  30

Tyr Asn Glu Lys Gln Ser Asn Glu Asn Phe Ala Lys Ala Val Asp Gln
        35                  40                  45

Met Thr Lys Ser Thr Val Thr Ala Ile Glu Ser Ile Lys Asp Thr Thr
    50                  55                  60

Val Thr Gly Ile Asn Ala Leu Leu Asn Met Asp Thr Leu Arg Asp Val
65                  70                  75                  80

Asn Ser Leu Ala Arg Glu Lys Ala Lys Asp Gln Asn Pro Ser Ser Gln
                85                  90                  95

Ala Lys

<210>  4
<211>  97
<212>  PRT
<213>  Artificial

<220>
<223>  P98 variant

<400>  4

Ala Ile Thr Leu Leu Glu Gly Ala Leu Tyr Gly Phe Phe Ala Val Thr
1               5                   10                  15

Gly Ile Leu Ile Gly Ser Phe Val Val Gly Glu Ile Val His Leu Tyr
            20                  25                  30

Asn Glu Lys Gln Ser Asn Glu Asn Phe Ala Lys Ala Val Asp Gln Met
        35                  40                  45

Thr Lys Ser Thr Val Thr Ala Ile Glu Ser Ile Lys Asp Thr Thr Val
    50                  55                  60

Thr Gly Ile Asn Ala Leu Leu Asn Met Asp Thr Leu Arg Asp Val Asn
65                  70                  75                  80

Ser Leu Ala Arg Glu Lys Ala Lys Asp Gln Asn Pro Ser Ser Gln Ser
                85                  90                  95

Lys

<210>  5
<211>  98
<212>  PRT
<213>  Artificial

<220>
<223>  P98 variant

<400>  5

Met Ala Ile Thr Leu Leu Glu Gly Ala Leu Tyr Gly Phe Phe Ala Val
1               5                   10                  15

Thr Gly Val Leu Ile Ala Ser Phe Ile Ile Gly Glu Ile Val His Leu
            20                  25                  30

Tyr Asn Glu Lys Gln Ser Asn Glu Asn Phe Ala Lys Ala Ile Asp Gln
        35                  40                  45

Met Ser Lys Ser Thr Val Thr Ala Ile Glu Ser Ile Lys Asp Thr Thr
    50                  55                  60

Val Thr Gly Ile Asn Ala Leu Leu Asn Met Asp Thr Leu Arg Asp Val
65                  70                  75                  80

Asn Ser Leu Ala Arg Glu Lys Ala Lys Asp Gln Asn Pro Ser Ser Gln
                85                  90                  95

Ala Lys

<210>  6

<211>  92
<212>  PRT
<213>  Artificial

<220>
<223>  P98 variant

<400>  6

Met Leu Ala Glu Ile Ala Leu Glu Gly Phe Leu Ala Ala Ile Gly Ala
1               5                   10                  15

Ile Ser Ala Val Phe Ile Ile Ala Glu Ala Ala His Leu Tyr Asn Glu
            20                  25                  30

Lys Ile Arg Asn Gln Ser Phe Gln Asn Ala Ile Asp Ala Met Ala Lys
        35                  40                  45

Ser Thr Val Val Ala Thr Glu Ser Ile Lys Asp Thr Thr Val Thr Gly
    50                  55                  60

Ile Asn Ala Leu Val Asn Met Asp Thr Leu Arg Asp Val Asn Asp Leu
65                  70                  75                  80

Ala Lys Ser Lys Ser Gln Asn Gln Pro Ala Gln Lys
                85                  90

<210>  7
<211>  92
<212>  PRT
<213>  Artificial

<220>
<223>  P98 variant

<400>  7

Met Ala Ile Asp Leu Thr Thr Ile Leu Tyr Ile Ile Ala Gly Leu Thr
1               5                   10                  15

Ala Leu Gly Val Gly Ala Asp Val Ile Leu Asn Tyr Leu Leu Lys Ser
            20                  25                  30

Glu Met Val Thr Ala Val Asp Ala Ile Asn Ala Thr Ala Gln Ser Ala
        35                  40                  45

Ile Asn Ala Ile Thr Ala Thr Thr Glu Thr Gly Ile Asn Ala Ile Ile
    50                  55                  60

Asn Met Ser Ile Leu Asn Asp Val Asn Gly Leu Ala Lys Lys Lys Val
65                  70                  75                  80

Asp Thr Asn Ser Thr Ser Gln Gln Ala Gln Lys Gln
                85                  90

## Description

### FIELD OF THE INVENTION

The present invention relates to a homomultimeric protein structure constituted by assembled monomers of the P98 protein of *Sulfolobus islandicus* rod-shaped virus 2 (SIRV2) or assembled monomer variants of said P98 protein. In a particular embodiment, this homomultimeric protein structure has a seven-fold rotational symmetry, and is found in an open conformation or closed conformation. A particular structure has the form of baseless 7-face pyramid. The invention also relates to a conjugate comprising or consisting of a homomultimeric protein structure of the invention to which one or more heterologous molecule(s) is attached. Furthermore, the invention also concerns a homomultimeric protein structure or a conjugate of the invention, inserted into or exposed at the surface of a lipid layer, of a vesicle or of a cell, and their uses thereof.

### BACKGROUND OF THE INVENTION

The vast majority of known archaeal viruses carries double-stranded (ds) DNA genomes and differ morphologically from dsDNA viruses of the two other domains of life, Bacteria and Eukarya (1). Moreover, the genomes of these viruses are also unique. The functions of more than 90% of putative genes cannot be identified due to the lack of homologues in the extant databases (1). Limited knowledge of the biology of archaeal viruses is one reason for our poor understanding of viral gene functions. Archaeal viral cycles have unusual features, which was recently demonstrated by the discovery of a unique virion release mechanism exploited by the *Sulfolobus islandicus* rod-shaped virus 2 (SIRV2) (2) and the *Sulfolobus* turreted icosahedral virus (STIV) (3). Among archaeal viruses, SIRV2 and STIV are the best studied with respect to host cell interactions. It was demonstrated that both are lytic viruses and that SIRV2 causes massive degradation of the host chromosome. Virion assembly takes place in the cytoplasm and coincides with the appearance of numerous prominent virus-associated pyramids (VAPs) on the host cell surface, which point outwards and rupture the S-layer. Shortly after their formation, VAPs open to the outside and create large apertures through which the virions escape from the cell (2, 3). The discovery of this unique virion release system raised questions regarding the nature of the VAP and mechanism of its formation.

The present application reports the isolation and purification of the VAPs formed by SIRV2 in *S*. *islandicus.* The inventors have shown that this structure is constituted by multiple copies of a sole protein, and that the assembly of this protein forms a very stable structure when submitted to extreme temperature and pH conditions. Moreover, the inventors have shown that this structure may, in an embodiment, be obtained, in various cell types, only by expressing the nucleic acid encoding the protein. Finally, the structure inserts into lipid layers or bilayers, with a part exposed out of the layer or bilayer. Therefore, the structure disclosed in the present application constitutes an excellent tool to study interaction and membrane dynamics, as well as to expose heterologous molecule(s) at the surface of lipidic structures or at the surface of cells.

### BRIEF DESCRIPTION OF THE DRAWINGS

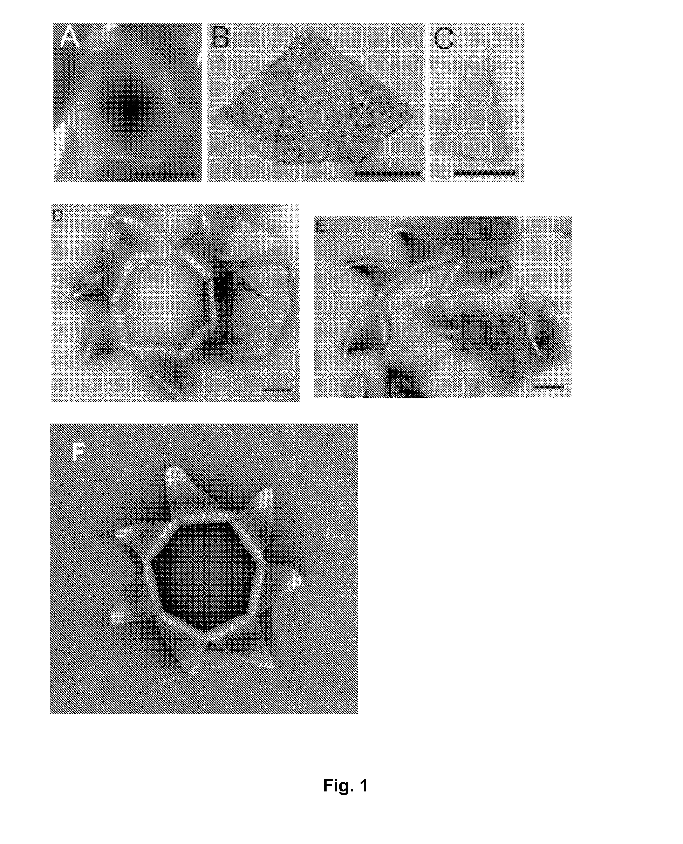

**Figure 1**: Negative contrast electron micrographs of isolated VAPs. (*A*) Top view and (*B*) side view of intact VAPs. (*C*) A single triangular face detached from a VAP. (*D*) Top view of a VAP in the open conformation. (*E*) Partially broken VAP in the open conformation (scale bars, 100 nm.) (F) Top view of a VAP in the open conformation.

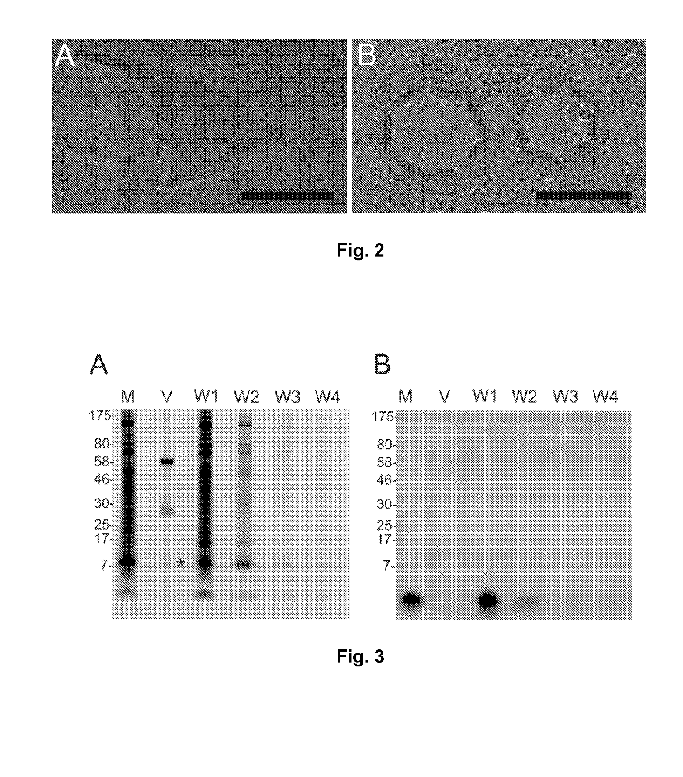

**Figure 2**: Cryo-electron micrographs of isolated VAPs. (*A*) Single curved face of a VAP. (*B*) Two VAPs from the top (scale bars, 100 nm.)
**Figure 3**: Isolation and constituents of VAPs. (A) SDS-PAGE of fractions obtained during immunoprecipitation and purification of VAPs. "M", crude membrane extract; "V", immunoprecipitated fraction; "W1-W4", four successive washes of the immunoprecipitate. Proteins were stained with SYPRO Ruby. (*B*) The same as in *A* but stained for phospholipids using Sudan Black. The P98 band in lane "V" is indicated with an asterisk (*). The positions of proteins with known molecular masses (in kDa) are indicated with bars.

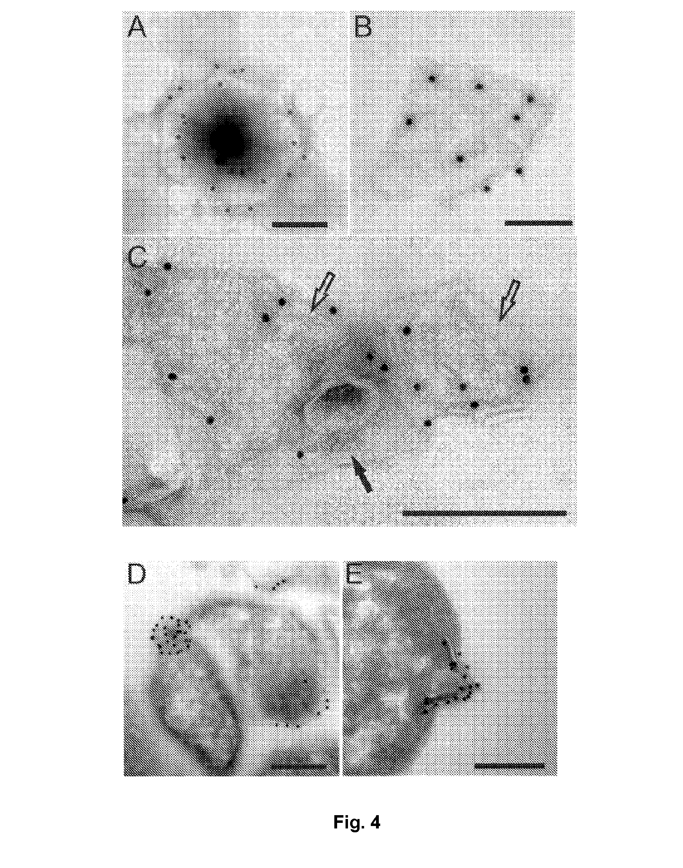

**Figure 4**: Negative contrast electron micrographs and P98 immunolabelling of VAPs. (*A, B*, and *C*) Purified VAPs seen from different angles. (*D* and *E*) Thawed cryosections through SIRV2-infected cells. The arrows in *C* indicate three individual VAPs, visualised from the side (empty arrows) and from below (filled arrows). (scale bars in *A-C*, 100 nm; scale bars in *D* and *E*, 200 nm).

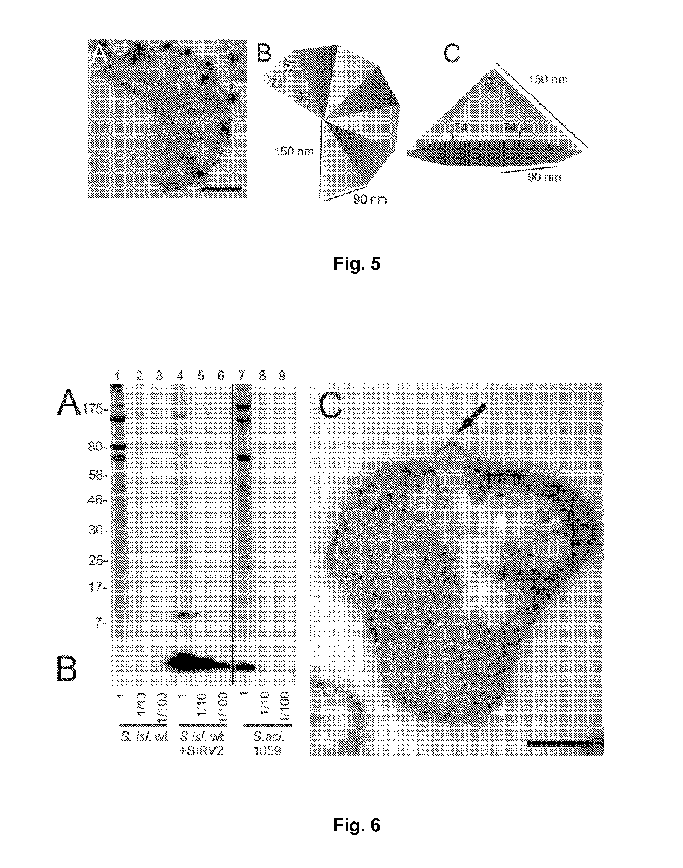

**Figure 5**: VAP structure. (*A*) Negative contrast electron micrograph and P98 immunolabelling of a partially disrupted VAPs. (*B*) Schematic representation of the image in *A*. (*C*) Schematic 3D reconstruction of the native VAP structure based on the images in *A* and *B*. The lengths and angles were measured from the image in *A.* (scale bar, 100 nm).
**Figure 6**: Heterologous expression of SIRV2-ORF98 in *S*. *acidocaldarius. (A, B)* SDS-PAGE of membrane fractions from the following samples: (1-3), uninfected *S*. *islandicus;* (4-6) SIRV2-infected *S. islandicus,* 10 h p.i; (7-9) *S*. *acidocaldarius* 1059 expressing SIRV2-ORF98. The samples were loaded without diluted and with dilutions of 1:10 and 1:100. The positions of proteins with known molecular masses (in kDa) are indicated with bars. The asterisk (*) shows the band containing P98. (A) Coomassie Blue-stained gel. (*B*) Western hybridisation of a duplicate of the gel in *A* with antibodies against SIRV2-P98. (*C*) Thin section through *S. acidocaldarius* 1059 expressing SIRV2-P98. The arrow indicates a VAP (scale bar, 200 nm).

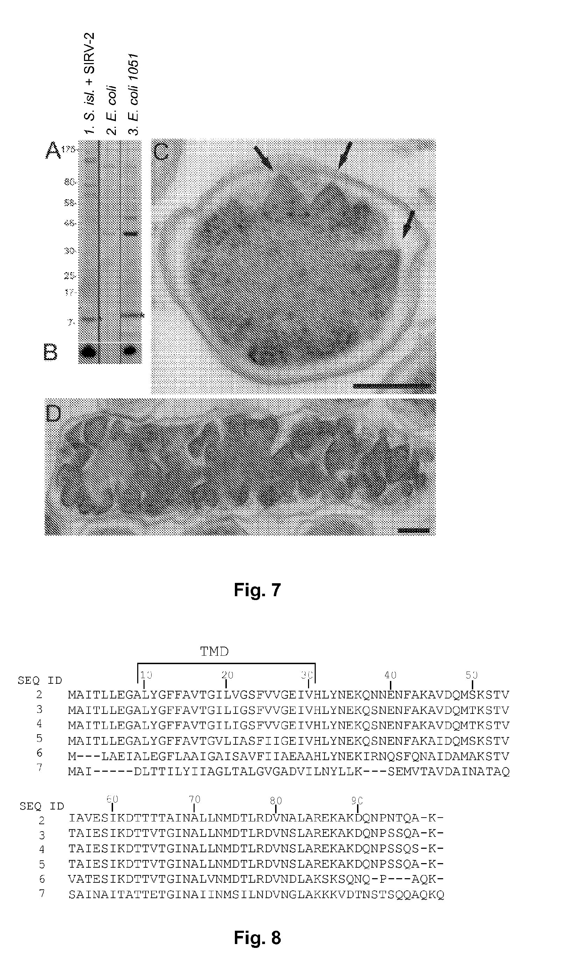

**Figure 7**: Heterologous expression of SIRV2-ORF98 in *E. coli. (A, B*) SDS-PAGE of membrane fractions from the following samples: (1) SIRV2-infected *S*. *islandicus,* 10 h.p.i.; (2) *E. coli;* (3) *E. coli* 1051 expressing SIRV2-ORF98. The positions of proteins with known molecular masses (in kDa) are indicated with bars. The asterisks (*) show the bands containing P98. (*A*) Coomassie Blue-stained gel. (B) Western hybridisation of a duplicate of the gel *A* with antibodies against SIRV2-P98. (*C*, *D)* Thin sections through *E. coli* 1051 expressing SIRV2-P98 (scale bars, 200 nm).
**Figure 8**: Alignement of the protein sequence of the P98 protein of SIRV2 and the protein sequence of five P98 variants (TMD: transmembrane domain).

### DETAILED DESCRIPTION

The invention relates to a homomultimeric protein structure constituted by assembled monomers of the P98 protein of *Sulfolobus islandicus* rod-shaped virus 2 (SIRV2) or assembled monomer variants of said P98 protein.

The expression *"homomultimeric protein structure"* means that the claimed structure consists of multiple identical protein monomers, *i.e*., that the structure results only from the assembly (or cluster) of identical protein monomers. In the described structure, the number of monomers is at least 100, at least 1000, at least 10000 or is comprised between 100 and 10000 or 100 and 1000. The expression *"homomultimeric"* encompasses not only the assembly of the structure by non covalent bonds (such as ionic bonds, hydrophobic interactions, hydrogen bonds and van der Waals forces) between identical monomers, but also the assembly of the structure by covalent bonds (such as peptide bonds) between identical monomers or the assembly of the structure by a combination of covalent and non covalent bonds between identical monomers. The homomultimeric protein structure does not as such comprise other protein or polypeptide components. In a particular embodiment, the homomultimeric protein structure does not comprise lipids or only a small proportion of lipids as compared to the number of monomers of the structure.

The protein monomer is selected among:
(a) the P98 protein of *Sulfolobus islandicus* rod-shaped virus 2 (SIRV2). The sequence of this P98 protein is given below and defined in SEQ ID NO:2: MAITLLEGALYGFFAVTGILVGSFVVGEIVHLYNEKQNNENFAKAVDQMSKSTVIAVE SIKDTTTTAINALLNMDTLRDVNALAREKAKDQNPNTQAK. The nucleic acid encoding the P98 protein is as defined in SEQ ID NO:1; and
(b) a variant of the P98 protein of SIRV2 (of SEQ ID NO:2), obtained by deletion, addition or substitution of one or more of its amino acid residues, provided that said variant is still able to build the homomultimeric protein structure. In a particular embodiment, the number of substituted, deleted and/or added amino acid residues is 1, 2, 3, 4, 5, 6, 7, 8, 9 or 10. In a particular embodiment, the variant has an amino acid sequence similarity of at least 50%, at least 60%, at least 70%, at least 80%, at least 85%, at least 90% or at least 95% with SEQ ID NO:2. In a particular embodiment, the variant is obtained by substitution, preferably conservative substitution, of 1 to 10 residues, preferably 1 to 5 residues, preferably 1, 2, 3, 4 or 5, in SEQ ID NO:2. The variant protein as defined above, is either natural (*i.e*., found in another virus) or artificial (*i.e*., not natural). Examples of P98 variants which may be used within the present invention have amino acid sequences defined in SEQ ID NOs: 3 to 7. The person skilled in the art may determine which amino acid residue(s) may be substituted or deleted, the site of insertion of residue(s) or the nature of the substituting residue(s), based on the alignment of the sequences SEQ ID NO:2 to SEQ ID NO:7 (Figure 8), and in particular based on the alignment of the sequences SEQ ID NO:2 to SEQ ID NO:5. In a particular embodiment, the P98 variant is one of SEQ ID NO:2 to 7 in which at least one cysteine residue(s) (preferably one) has been inserted within the protein or at one of the extremities of the protein, provided this variant is still able to build the homomultimeric protein structure. The ability for a P98 variant to form the homomultimeric protein structure may be easily tested by expressing the nucleic acid encoding this variant from a plasmid, which is transfected in *Sulfolobus* or in *Escherichia coli* and by determining whether the transfected cells produce or not the homomultimeric protein structure of the invention.

The homomultimeric protein structure is built naturally by assembly of the P98 protein of SIRV2 (or its variants), *i.e*., that the P98 protein of SIRV2 is sufficient alone to form the homomultimeric protein structure. This is confirmed by the examples which show that the homomultimeric protein structure is formed not only in an archaeon *(Sulfolobus acidocaldarius)* but also in particular in the bacteria *Escherichia coli.* Therefore, the homomultimeric protein structure may be obtained by expression of the nucleic acid encoding the P98 protein of SIRV2 (or its variants) in a cell or cell culture. In a particular embodiment, the expression of the nucleic acid encoding the P98 protein of SIRV2 (or its variants) is carried out in archaeon (such as a *Sulfolobus strain,* for example *Sulfolobus acidocaldarius),* in a prokaryote (such as bacteria, for example *E. coli)* or in an eukaryote cell, such as a yeast, a plant cell, an animal cell, preferably a mammalian cell, more preferably a human cell. The expression of the nucleic acid encoding the P98 protein of SIRV2 (or its variants) may be obtained from an expression vector (such as a plasmid) or from the cell genome in which the nucleic acid is inserted, possibly after codon optimization to improve expression in selected cells. Alternatively, the homomultimeric protein structure may also be obtained *in vitro* in solution in appropriate conditions by assembly of the P98 proteins (or variants).

The ability to give rise to a homomultimeric protein structure by the self-assembly of the P98 protein of SIRV2 (or its variants) is a property of the protein sequence of the P98 protein of SIRV2 (or its variants), *i.e*., that the P98 protein of SIRV2 (or its variants) spontaneously self-assembles to adopt this homomultimeric protein structure. In a particular embodiment, this homomultimeric protein structure has a seven-fold rotational symmetry.

This homomultimeric protein structure consists of seven isosceles triangles, the area of which is constituted by assembled monomers of the P98 protein of SIRV2 or assembled monomer variant of the P98 protein, the base of each of these isosceles triangles forming the sides of a regular heptagon. The homomultimeric protein structure has thus a heptagonal basis (formed of P98 proteins), each side of this heptagon being also the base of the isosceles triangles.

The heptagonal basis of the structure is empty or hollow. The heptagonal basis is regular, *i.e*., all sides and all angles are slightly equal, *i.e*., each angle of the heptagon is comprised between 125 and 132° and is especially about 128.5° ± 2°. Though all sides of the heptagon are substantially identical in length, the length of all sides of the regular heptagon may vary together thus enabling variatioin of the size of the entire structure. Accordingly, the size of the sides of the heptagon is comprised between 15 and 175 nm, preferably between 20 and 100 nm.

Each isosceles triangle is formed of P98 proteins, and in contrast to the heptagonal basis, its area is completely constituted of assembled P98 proteins, *i.e*., the isosceles triangles are full. In a particular embodiment, the angle of the main vertex (*i.e*., the angle formed by the meet of the two equal sides of the triangle) of each isosceles triangles is comprises between 29 and 35° and is about 32° ± 3° (the two remaining angles are identical and are thus about 74° ± 8°).

It is noteworthy that the geometry of the homomultimeric protein structure does not change over the course of formation of the structure, and over the growth of the structure which is achieved via gradual expansion of the triangular faces.

In a first embodiment, the homomultimeric protein structure is a pyramid with 7 identical isosceles triangular faces and with a hollow base, *i.e*., is a baseless pyramid with 7 identical isosceles triangular faces (Figure 5C). In this conformation, the vertices of the isosceles triangular faces are connected or linked to each other, and acccordingly close the structure. Thus, one of the equal sides of each isosceles triangular face is connected, two by two, to the equal side of the immediately adjacent isosceles triangular face. The structure in pyramid can also be viewed as a closed conformation. In a particular embodiment, the baseless 7-face pyramid has a diameter ranging from 30 to 250 nm, preferably 40 to 200 nm. Whatever the diameter or size of the homomultimeric protein structure, its base keeps its regular heptagonal shape and isosceles triangular faces. In a particular embodiment, the angle formed between each triangular face and the empty heptagonal basis is comprised between 50 and 56 and is about 53.5° ± 2.

In another embodiment, the homomultimeric protein structure has an open conformation, *i.e*., that the vortex of each isosceles triangle is free (not connected to another vertex). Because of its particular shape, the homomultimeric protein structure in an open confirmation may have a diameter larger than the diameter of the baseless 7-face pyramid, that can exceed 250 nm. An example of a homomultimeric protein structure of the invention in an open conformation is disclosed in Figure 1F.

The homomultimeric protein structure of the invention, whatever its conformation, but preferably under the form of baseless 7-face pyramid, is very stable, when submitted to conditions comprising from moderate to extremely high temperatures and/or from neutral to extremely acidic pH. Thus, the homomultimeric protein structure is stable at a temperature as high as 80°C and/or stable at a pH as low as pH2.

The ease to produce the homomultimeric protein structure of the invention combined to its stability at extreme temperature and/or pH, makes this structure an excellent tool to study protein-protein interaction in membranes such as cell membranes, such as cell membranes, as well as to modify the structure of the cell membrane and to expose heterologous molecule(s).

Thus, the invention also relates to a conjugate comprising or consisting of a homomultimeric protein structure of the invention, preferably the baseless 7-face pyramid, to which one or more heterologous molecule(s) is attached. By *"one or more",* it is meant that either only one molecule is attached to the homomultimeric protein structure, that many copies of the same heterologous molecule are attached to the homomultimeric protein structure or in contrast that distinct (different) heterologous molecules are attached, each in one copy or in many copies, to the homomultimeric protein structure. The term *"molecule"* encompasses drug, protein, polypeptide, nucleic acid (DNA, RNA, siRNA) or chemical compound. By *"heterologous",* it is meant any molecule as defined herein which is not the P98 protein or a variant as defined in the present application. The attachment of the heterologous molecule to the homomultimeric protein structure (in particular the P98 protein or variant) is either covalent (peptide bonds) or non-covalent (ionic bonds, hydrophobic interactions, hydrogen bonds and van der Waals forces). In the absence of cysteine residue in the protein sequence of the P98 protein, the heterologous molecule may be attached, by disulfide bonds, to a P98 variant (as defined above) in which at least one cysteine residue(s) (preferably one) has been inserted within the protein or at one of the extremities of the protein. Thus, the homomultimeric protein structure of the invention is used as an anchor for the one or more heterologous molecule(s). In a particular embodiment of the invention, not all P98 proteins (or variants), constituting the homomultimeric protein structure of the invention, bear heterologous molecule(s). Thus, in a particular embodiment, less than 50%, less than 40%, less than, 30%, less than 20% or less than 10% of the P98 proteins (or variants) bear heterologous molecule(s). In another particular embodiment, when the homomultimeric protein structure is a baseless 7-face pyramid, the heterologous molecule(s) is attached to the apex of the pyramid, in particular on the part of the pyramid which is exposed out of the lipid layer or bilayer, in particular out of the cell lipid membrane. The heterologous molecules may be attached to the homomultimeric protein structure (in particular the P98 protein or variant) either before the assembly of the P98 proteins (or variants), during the assembly of the P98 proteins (or variants), or once the homomultimeric protein structure is assembled. In particular, the high stability of the homomultimeric protein structure of the invention enables the attachment of the heterologous molecule(s) once the structure is assembled, in various conditions, without denaturing or altering the resulting homomultimeric protein structure.

Thus, the invention also relates to the use of the homomultimeric protein structure of the invention, preferably the baseless 7-face pyramid structure, or a conjugate as defined herein, to insert into or to expose said homomultimeric protein or conjugate at the surface of a lipid layer, in particular of a lipid bilayer. Thus, the invention relates to a lipid layer, in particular a lipid bilayer, comprising inserted into the layer (or bilayer) or exposed on its surface, a homomultimeric protein structure or one or several conjugate(s) of the invention. By *"one or several conjugate(s)",* it is meant that either only one conjugate is inserted into or exposed on the lipid layer (or bilayer), that many copies of the same conjugate are inserted into or exposed on the lipid layer (or bilayer) or in contrast that distinct (different) conjugates are inserted into or exposed on the lipid layer (or bilayer), each in one copy or in many copies. The difference between conjugates originate especially from the nature, composition or number of heterologous molecule(s) anchored or associated on the homomultimeric protein structure.

The lipid layer or lipid bilayer may be artificial or may be extracted from a cell membrane as a fragment.

In a particular embodiment, the homomultimeric protein structure of the invention, preferably the baseless 7-face pyramid structure, or conjugate of the invention, is inserted into or is exposed at the surface of a model lipid layer, *i.e*., an artificially created lipid layer. Well known model lipid layers are black lipid membranes (BLM), supported lipid bilayers (SLB), the tethered bilayer lipid membrane (t-BLM) and vesicles.

Thus, the invention relates to a membrane microarray, in particular a cell membrane microarray, which comprises a solid support and a fluid supported lipid bilayer, wherein said bilayer comprises the homomultimeric protein structure or conjugate of the invention. Examples of solid supports are surfaces composed of or based on materials such as metal, metal oxides or mineral materials, especially gold, glass, diamond, silicon, silicon dioxide (SiO₂), silicon nitrite, tantalum pentoxide (Ta₂O₅), titanium dioxide (TiO₂), titanium nitrite, titanium carbide, platinum, tungsten, aluminium or indium oxide, or mixtures thereof. In a particular embodiment, the surface is functionalized, *i.e*., coated with a mixture of molecules able to interact, preferably in a covalent manner, with the elements present in the lipid bilayer (such as phospholipids or specific ligands).

The invention also concerns biomimectic artifical membrane comprising a semipermeable membrane for supporting a lipid membrane and a lipid membrane comprising a plurality of lipid molecules arranged in a layer or bilayer, wherein said layer or bilayer comprises the homomultimeric protein structure or conjugate of the invention. In a particular embodiment, the supporting membrane is a porous polymer, silicon or graphene membrane.

In a particular embodiment, the invention also relates to a vesicle, in particular artificial vesicle, such as a micelle, bicelle, nanodisc or liposome, comprising inserted in the layer (or bilayer) or exposed on its surface a homomultimeric protein structure or conjugate of the invention. In a particular embodiment, when said homomultimeric protein structure is a baseless 7-face pyramid or a conjugate obtained from a baseless 7-face pyramid, the basis of the pyramid is in the lipid layer or bilayer and the apex of the pyramid is outside of the lipid layer or bilayer.

A lipid layer, lipid bilayer or model lipid layer of the invention may be used to assay *in vitro* molecule-molecule interactions, especially protein-protein interactions, in particular with the heterologous molecule(s) attached to the homomultimeric protein structure of the conjugate. Thus, the application relates to a process to assay *in vitro* molecule-molecule interactions, especially protein-protein interactions, comprising: (a) putting a lipid layer, in particular a lipid bilayer, comprising inserted into the layer (or bilayer) or exposed on its surface, a homomultimeric protein structure or conjugate(s) of the invention in contact with a solution to assay (comprising unidentified molecules); and (b) determining whether a molecule of this solution is able to interact with the homomultimeric protein structure or with the heterologous molecule(s) of the conjugate(s) of the invention. The invention also relates to a process to assay *in vitro* molecule-molecule interactions, especially protein-protein interactions, comprising: (a) putting a lipid layer, in particular a lipid bilayer, comprising inserted into the layer (or bilayer) or exposed on its surface, a homomultimeric protein structure or conjugate(s) of the invention in contact with a known molecule; and (b) determining whether this known molecule is able to interact with the homomultimeric protein structure or with the heterologous molecule of the conjugate of the invention. These two processes are particularly interesting to assay interactions between a receptor, particularly a transmenbrane receptor and known or unidentified ligands. The homomultimeric protein structure of the invention being highly stable, it is possible to carry out step (b) by isolating the homomultimeric protein structure from the layer, bilayer or membrane cell and by analysing, with conventional techniques, the molecule(s) which interact(s) with this structure or with the heterologous molecule of the conjugate.

The invention also relates to the use of a lipid layer, lipid bilayer or model lipid layer of the invention to analyze the genome, a transcriptome, a proteome or a metabolome, using conjugate(s) of the invention wherein the heterologous molecule is respectively a DNA probe, a protein-based prey or a drug candidate prey. By *"transcriptome",* it is meant a set of RNA, including mRNA, rRNA, tRNA, and other non-coding RNA, in particular mRNA. By *"proteome",* it is meant a set of proteins and/or polypeptides. By *"metabolome",* it is meant a set of metabolites such as metabolic intermediates, hormones and other signalling molecules, and secondary metabolites. Transcriptome, proteome and metabolome may be determined for a cell, a cell population, a cell type (organ-specific, tumor cells), a pathogen, a virus, a sample (plasma, blood), ...

Thus, the invention concerns a method (or process) to analyze the genome or a transcriptome of a cell, a cell population, a cell type or a sample, comprising:
(a) putting a lipid layer, in particular a lipid bilayer, comprising inserted into the layer (or bilayer) or exposed on its surface, conjugate(s) of the invention wherein said heterologous molecule is a nucleic acid (*e.g*., DNA) probe, in contact with a DNA or RNA preparation (in particular a mRNA preparation) from a cell, a cell population, a cell type or a sample; and
(b) determining the RNA molecules, that interact (*e.g*., hybridize) with the probes of the conjugate(s), to define the genome or the transcriptome of said cell, cell population, cell type or said sample, or to define a fingerprint thereof.

Thus, the invention is also directed to a method (or process) to analyze a proteome of a cell, a cell population, a cell type, a pathogen, a virus or a sample, comprising:
(a) putting a lipid layer, in particular a lipid bilayer, comprising inserted into the layer (or bilayer) or exposed on its surface, conjugate(s) of the invention wherein said heterologous molecule is a protein-based prey or drug candidate prey, in contact with a protein preparation from a cell, a cell population, a cell type, a pathogen ,a virus or a sample; and
(b) determining the proteins, that interact with the preys of the conjugate(s), to define the proteome of said cell, cell population, cell type, a pathogen, virus or sample, or to define a fingerprint thereof.

Thus, the invention is also directed to a method (or process) to analyze a metabolome of a cell, a cell population, a cell type or a sample, comprising:
(a) putting a lipid layer, in particular a lipid bilayer, comprising inserted into the layer (or bilayer) or exposed on its surface, conjugate(s) of the invention wherein said heterologous molecule is a protein-based prey or drug candidate prey, in contact with a preparation from a cell, a cell population, a cell type or a sample; and
(b) determining the metabolites, that interact with the preys of the conjugate(s), to define the metabolome of said cell, cell population, cell type or sample, or to define a fingerprint thereof.

Another application concerns the vesicle, micelle, bicelle, nanodisc or liposome as defined herein which may be used to transport and possibly to expose *in vivo* the heterologous molecule(s) attached to the homomultimeric protein structure of the conjugate. Thus, the application also relates to a vesicle, micelle, bicelle, nanodisc or liposome as defined herein, for use as a medicament. In particular, the vesicle, micelle, bicelle, nanodisc or liposome comprising inserted into its layer (or bilayer) a conjugate as defined herein, more particularly a conjugate comprising a biologically active molecule as a heterologous molecule.

The invention also relates to a cell (or cell population), in particular an isolated cell or a cultured cell, expressing, in or on one of its membrane, the homomultimeric protein structure or the conjugate of the invention. In a particular embodiment, the cell is a non-archaea cell. In a particular embodiment, the cell is a prokaryotic cell (such as bacteria, for example *E. coli)* or an eukaryotic cell, such as a yeast, a plant cell, an animal cell, preferably a mammalian cell, more preferably a human cell. These cells, in particular cells expressing in their membrane or exposing on the surface of their membrane a conjugate of the invention, can be used to *in vitro* study the cell membrane, the cell membrane dynamics or the interactions of cell membrane proteins. These cells may also be used to transport and to expose *in vivo* the heterologous molecule(s) attached to the homomultimeric protein structure (as conjugate). Thus, the application also relates to a cell as defined in the invention for use as a medicament. In particular, the cell comprising inserted in or one of its membrane a conjugate as defined herein, more particularly a conjugate comprising a biologically active molecule as a heterologous molecule.

### EXAMPLES

### A) Materials and Methods

**Virus and host strains**. The stock of the SIRV2 virus was prepared as described in ref. 2. The growth of *S. islandicus* LAL/14 and virus infection were also described in ref. 2.

**VAP isolation**. *S*. *islandicus* LAL 14/1 cells infected with SIRV2 and uninfected controls were harvested 10 h.p.i (hour post infection). The cells were disrupted using a French press, and the membranes were collected by centrifugation at 100,000 g as described in ref. 4. VAPs were isolated from the membrane fraction using anti-P98 antibody. The customised P98 polyclonal peptide antibody was raised in rabbit against a peptide corresponding to the C-terminal region of P98 and affinity purified using this antigen (Eurogentec). The specificity of the antibody was tested on a Western blot. The antibodies were coupled to magnetic beads covered with protein A (Dynabeads Protein A, InvitrogenTM) according to the manufacturer's protocol using non-denaturing elution. Alternatively, the membrane fraction was washed in 0.5% N-lauroylsarcosine (Sigma) and centrifuged at 100,000 g, and the pellet was used for immunoprecipitation.

**Protein and lipid analysis**. The protein fractions were loaded onto a 4-12% polyacrylamide Bis-Tris gradient gel (invitrogenTM) using 2-(N-morpholino) ethanesulphonic acid SDS running buffer (invitrogenTM), and the proteins were visualised using either SYPRO Ruby (invitrogenTM) or Coomassie-based Instant BlueTM (Expedeon) staining. The presence of lipids in the samples was detected by overnight staining in Sudan Black (Sigma-Aldrich) according to the manufacturer's protocol. MS analysis was performed as described in ref. 4. For Western hybridisation, proteins were transferred onto a PVDF membrane. P98 was visualised using 1:10000 dilution of the anti-P98 antibody and peroxidase-coupled goat anti-rabbit IgG (Sigma-Aldrich) and the SuperSignal® West Pico Chemiluminescent Substrate (Thermo Scientific) on Amersham Hyperfilm™ ECL (GE Healthcare).

**Negative contrast EM and immunolabelling of isolated VAPs**. Purified VAPs were absorbed to glow discharged carbon-coated grids and negatively stained for 1 min with 2% (wt/vol) uranyl acetate. For immunocytochemistry, after absorption on the grids, the samples were incubated in PBS with 1% BSA and labelled with the anti-P98 peptide antibody. The antibody-labelled protein was detected using 10-nm Protein A-colloidal gold (CMC, The Netherlands). All samples were observed on a Jeol 1200EX-II operated at 80 kV. Images were recorded and measured on a MegaView or KeenView camera (SIS, Japan) using the ITEM software version 5.0 (SIS, Japan).

**Cryo-EM of isolated VAPs**. Purified VAPs were spread on glow discharged Quantifoil R2/2 grids (Quantifoil Micro Tools GmbH, Germany) and cryofixed in liquid ethane. The specimens were transferred to a Gatan 626 DH cryoholder (Gatan, USA) and examined on a JeoI2010F electron microscope (JeoI, Tokyo, Japan) operating at 200 kV. Images were recorded under low-dose conditions on a Gatan Ultrascan 4000 with Digital Micrograph (Gatan, USA) version 1.83.842.

**Immunolabelling of thawed cryosections**. SIRV2-infected *S. islandicus* cells were fixed 10 h.p.i. with 4% formaldehyde in 0.1 M HEPES buffer, pH 6, for 5 h at 4°C. The cells were pelleted by low speed centrifugation, embedded in 10% gelatine, cut into small blocks and infiltrated in 2.3 M sucrose at 4 °C. The blocks were mounted on aluminium pins and frozen in liquid nitrogen. Sections were cut with a nominal feed of 70 nm using a 35° angle diamond knife (Diamtome, Biel, Switzerland) and a FC6 microtome (Leica Microsystems, Vienna, Austria). To pick up the ultrathin cryosections, a 1:1 mixture of 2.3 M sucrose and 2% methylcellulose was used. Thawed sections were placed on formvar and carbon-coated copper grids, and sections were blocked in 1% BSA and labelled with the anti-P98 peptide antibody and 10-nm Protein A-colloidal gold (CMC, Utrecht, The Netherlands). Images were recorded on a KeenView camera (SIS, Japan) using the ITEM software version 5.0 (SIS, Japan).

**Resin embedding of *Sulfolobus* cells.** *S. acidocaldarius* MR31 pSVA1059 and SIRV2-infected *S. islandicus* were fixed with 2.5% (wt/vol) glutaraldehyde in 0.1 M HEPES buffer, pH 6 and 7, respectively. Post-fixation, dehydration, embedding in epoxy resin, sectioning and imaging with TEM were performed as described in ref. 2.

**High pressure freezing and freeze substitution of** ***E.coli.** E.coli* cells were taken up in capillary tubes (Leica, Vienna, Austria) as described in ref 9. The filled tube was placed into the cavity of a brass planchette (Agar Scientific, Stanstad, UK), filled with 1-hexadecen, and immediately frozen with a HPM 010 (BaITec, now Abra Fluid AG, Widnau, Switzerland). Freeze-substitution was performed in anhydrous acetone (EMS, Hatfield, USA) containing 2% osmium tetroxide (Merck, Darmstadt, Germany) and 2% water. Substitution was performed at -90 °C for 24h, and at -60 °C and -30 °C for 8h each in an automated freeze substitution device (Leica AFS, Leica Microsystems, Vienna, Austria). Afterwards the temperature was raised to 0 °C and the samples washed with dry acetone and embedded stepwise in Epon and polymerized at 60°C for 48h.

### Plasmid constructs and transformation of S. acidocaldarius and E. coli.

For the overexpression of P98 in *S. acidocaldarius* M31, SIRV2-ORF98 (SEQ ID NO:2) was amplified from SIRV2 genomic DNA and cloned into the *lacS* gene locus in the pSVA5 plasmid (10) using the Ncol and BamHI sites. SIRV2-ORF98 and the *araS* promoter were transferred from pSVA5 to the pCMaILacS plasmid (5) using the Ncol and Eagl sites, which yielded pSVA1059. *S. acidocaldarius* M31 was transformed with pSVA1059. The preparation of competent cells, methylation of the plasmid, and electroporation were carried out as described in ref 1. The electroporator used was the Gene Pulser Xcell (Bio-Rad) with 1 mm cuvettes at 1500 V, 600 Ω and 25 µF. The cells were regenerated in Brock medium containing 0.1% enzymatically hydrolysed casein (tryptone, BD Biosciences) and 0.2% dextrin for 30 min at 75 °C. The cells were then streaked onto selective Gelrite® (Sigma) plates lacking uracil. After 5 days, a preculture was grown under selective conditions from a single colony; 50 mL of medium containing inducer (0.2% maltose) was then inoculated with 1 mL preculture and grown until an OD600 of 1 was reached. For the overexpression of SIRV2-ORF98 in *E. coli* Rosetta(DE3)pLys (Novagen), the same gene was amplified from SIRV2 genomic DNA and cloned into the T7 promoter-driven expression vector pSA4 (6) using the Ncol and BamHI sites. The pSVA1051 vector contains an IPTG-inducible promoter that was used for the expression a C-terminal his-tagged protein.

### B) Results

**VAP isolation**. The shape of the pyramidal egress structures VAPs, which were reported to be found on the surface of SIRV2-infected *S. islandicus* cells (2), suggested that they might represent individual structures. To test this hypothesis, we analysed the membrane fraction of infected cells that were disrupted using a French press. By transmission electron microscopy (TEM); it was observed for the first time numerous individual particles which had pyramidal shapes and heptagonal bases. The resemblance of their shape to that of VAPs observed *in vivo* supported the notion that VAPs represent autonomous structures. We isolated the particles using antibodies against protein 98 (P98) encoded by SIRV2, which we had previously postulated to be a constituent of the VAP (4). Polyclonal antibodies were fused to magnetic beads and used to bind VAPs in crude membrane extracts of infected cells collected 10 hours post infection (h.p.i.), which corresponds to the start of virion release. It was possible to precipitate and purify the VAPs in this manner. In the purified preparation, no structures other than VAPs were observed, except for a small proportion of S-layer fragments from the host cells. Moreover, the majority of the isolated structures appeared to be intact and similar in shape to the VAPs on the surface of the infected cells (2) (Fig. 1 *A, B).* The intact purified VAPs could either be observed from the top, which revealed the seven-fold rotational symmetry of the structure (Fig. 1*A*), or from the side, in which case only three to four of the seven triangular faces of the VAPs were visible (Fig. 1*B*). Along with the intact VAPs, in the purified preparation single triangular subunits were also found to be detached from the structure (Fig. 1*C*). In addition, a small proportion of the VAPs were present in an "open" conformation (Fig. 1*D* and *E*). Characteristically, in the latter case, the seven faces of the VAPs were not strictly triangular but rather were slightly curved (Fig. 1*D* and *E*) and resembled the structure of the VAP *in vivo* after it opens. To exclude the possibility that this curvature was an artefact of sample preparation for negative staining, the VAPs were analysed using cryo-EM. The faces of the open VAPs that were fixed in vitreous ice were also curved (Fig. 2*A*). The VAPs were best observed from the top, which revealed the heterogeneity of the diameter of the VAPs (Fig. 2*B*).

**VAP constituents**. The ability to isolate intact VAPs allowed studying their constituents. The protein composition of the VAPs was analysed by SDS-PAGE followed by SYPRO Ruby staining (Fig. 3*A*). The three visible bands in the purified VAP preparation were identified by Matrix-assisted laser desorption/ionisation (MALDI)-time-of-light (TOF) mass spectrometry (MS) and MS/MS analyses. The two larger proteins represented the light and heavy chains of the anti-P98 antibody used to purify the VAPs. The smallest protein (approximately 10 kDa, indicated by an asterisk) was identified as the product of SIRV2-ORF98 (SEQ ID NO:2) (Fig. 3*A*). To test the presence of phospholipids in the purified VAPs, a duplicate of the gel shown in Fig. 3A was stained using Sudan Black (Fig. 3*B*). Phospholipids could be observed in the membrane fraction and the first two washes of the precipitate but were not detectable in the purified VAP preparation.

**Immunolabelling of VAPs**. The localisation of SIRV2-P98 in the VAPs was verified by immuno-electron microscopy using polyclonal P98 antibodies and 10-nm protein A-gold particles (Fig. 4). The purified VAPs were labelled evenly across the structure, without concentration of the label in a particular area (Figs. 4*A*, *B* and *C*). As a control, similar labelling was performed on a crude membrane fraction of infected cells, which, in addition to VAPs, contained fragments of S-layers, membranes and virions. In this case, only VAPs were specifically labelled. Another control was performed by labelling purified VAPs with pre-immune serum and 10-nm protein A-gold particles. In this case, very low background labelling was observed. The specific localisation of P98 in the VAPs was also confirmed in *S*. *islandicus* cells infected with SIRV2 h.p.i. Thawed ultrathin cryo-sections of chemically fixed cells were immunolabelled. VAPs in the ultra-thin sections had either an heptagonal appearance if the section was in the plane parallel to the cell surface and the base of the VAP or a triangular appearance if the sections were in other planes (2). As a result of labelling, the gold particles were arranged in either a quasi-heptagonal shape (Fig. 4*D*) or along two sides of a VAP triangle (Fig. 4*E*). As a control, thawed ultra-thin cryo-sections of uninfected *S*. *islandicus* cells were labelled in a similar manner, and no labelling of any specific structure was observed.

**VAP structure**. Electron microscopy of isolated VAPs was used to produce a detailed description of their structure. Images of 150 intact or partially disrupted VAPs were measured. Independent of the sizes, the geometry of all VAPs was identical and represented a baseless pyramid with a heptagonal perimeter. Each of the seven faces of the pyramidal structure was an isosceles triangle, *i.e*., two sides were equal in length. The two angles at the base of the pyramid were 74°± 8°, and the one at the tip was 32° ± 3°. The structure shown in Fig. 5A represents a VAP in the closed form that was disrupted only along one seam between two of the seven faces. A two-dimensional schematic of the VAP is schematically presented in Fig. 5B, and folding of this sequences results in the three-dimensional structure shown in Fig. 5*C*. The structure perfectly matches all of the electron microscopic images of the VAPs taken from different angles. The data derived from isolated VAPs allowed concluding that the VAP is a baseless, hollow, pyramidal structure (Fig. 5C). This interpretation agrees with earlier reports of the structure of the VAP *in vivo.* Thin sections of infected cells suggest that cytoplasm is present in the interior of the VAP (Fig. S2A) (2).

The VAPs in the analysed preparation were isolated from a quasi-synchronous population of host cells 10 h.p.i., and although they had identical geometries, they differed significantly in size. The lengths of the sides of the regular heptagonal perimeter of the VAP were measured using 150 images of isolated closed structures and ranged from about 15 nm to about 175 nm. These structures were grouped into four size classes: (I) 0-50, (II) 50-100, (III) 100-150 and (IV) 150-200 nm. The following percentages of VAPs in each class were found: class I, 22%; class II, 25%; class III, 47%; and class IV, 7%. The most likely cause of the observed size distribution and the low percentage of class IV structures appears to be the proximity of the latter to the open state. This suggestion is based on the measurements of the side lengths of 20 open VAPs, of which 95% were in the range of 125-180 nm.

**Overexpression of SIRV2-ORF98 in** ***Sulfolobus acidocaldarius**.* Although P98 was found to be the sole constituent of the VAPs, it remained unclear whether it was sufficient for the formation of the pyramidal structure. To examine this possibility and to exclude the involvement of any other SIRV2-encoded factors in VAP assembly, SIRV2-ORF98 was expressed heterologously in the hyperthermophilic archaeon *Sulfolobus acidocaldarius.* This member of the genus *Sulfolobus* is resistant to SIRV2 and does not carry any extrachromosomal element, or any genes homologous to those of SIRV2. For expression, SIRV2-ORF98 was cloned under the control of a maltose-inducible promoter in the pCMalLacS plasmid (5). The level of expression, estimated by Western hybridisation, was about 100-times lower than that produced by SIRV2 infection of *S*. *islandicus* (Figs. 6A and 6B). Remarkably, the analysis of ORF98-expressing *S. acidocaldarius* cells by electron microscopy showed the presence of VAPs on the cell membranes (Fig. 6*C*). The VAPs perforated the S-layer and were similar in size and geometry to the VAPs of SIRV2-infected *S. islandicus* cells. The low number of observed VAPs, i.e., about 1 in 100 cells, corresponded to the low level of ORF98 expression in *S. acidocaldarius.* It is noteworthy that only closed VAPs were observed in the heterologous system, whereas in the natural virus-host system VAPs in both closed and open states are present (2,3)..

**Overexpression of SIRV2-ORF98 in** ***Escherichia coli**.* To exclude the possibility that some proteins common to members of the genus *Sulfolobus* are involved in VAP formation and to further test the self-assembly capacity of P98, the SIRV2-ORF98 gene was expressed in *E. coli.* The gene was cloned under a *lacs* promoter into the pSA4 plasmid (6). Expression was confirmed by Western hybridization, and the efficiency of P98 production was comparable to that of SIRV2-infected *S. islandicus* cells (Figs. 7A and B). The presence of the recombinant P98 was detected primarily in the membrane fraction. Surprisingly, P98 was not only produced in *E. coli* but was also capable of forming VAPs in these cells. Electron microscopy analysis of thin sections of P98-expressing cells of *E. coli* at 3, 7 and 21 hours after induction, revealed the presence of many pyramidal structures on the inner membrane, which protruded into the periplasmic space of the cell (Fig. 7*C* and *D*). The VAPs appeared to be similar in size and shape to those observed on the cell surface of SIRV2-infected *S. islandicus* and SIRV2-ORF98 expressing *S. acidocaldarius* (Fig. 7*C*). Only closed VAPs were observed. Three hours after induction, the majority of cells contained VAPs; however, the cells stopped growing almost immediately after induction, and the proportion of VAP-containing cells did not change significantly in the following 21 hours. In addition, the number of observed VAPs per cell did not change over time. The number of VAPs was generally very high and correlated with the expression levels of the viral gene. In most sections, more than 30 VAPs were observed per cell (Fig. 7*D*). The formation of VAPs seemed to increase the surface area of the inner membrane, and the distance between the outer and inner membranes (Figs. 7*C* and *D*).

### DISCUSSION

The discovery of the exceptional VAP-based virion egress system in Archaea (2, 3) has drawn attention to the underlying molecular machinery. The present finding that the VAPs represent separate structural units that can be isolated and purified from the membrane fraction of infected cells is crucial for understanding the nature and formation of these remarkable structures. Purification of VAPs was achieved by immunoprecipitation using antibodies against protein 98 of SIRV2, which was previously postulated to be a component of VAPs (4).

The VAPs were isolated and purified from a quasi-synchronised culture of SIRV2-infected *S*. *islandicus* at the start of virion release. The majority of purified VAPs appeared to be intact. Each VAP was composed of seven faces of isosceles triangles that together formed a baseless pyramid. In all cases, the angles of the faces were 74°± 8° and 32°± 3°. However, the diameters of the VAPs were heterogeneous. This heterogeneity most likely reflects the dynamics of VAP development in the analysed cell culture. The observations suggest that VAP geometry does not change over the course of formation and that VAPs grow via gradual expansion of the triangular faces.

In addition to VAPs in the natural closed conformation, the purified preparation contained VAPs that were in the open state (Fig. 1). These VAPs might have originated from perforated cells. Indeed, the population of infected cells that was used for VAP isolation contained a small proportion of perforated cells with open VAPs. Alternatively, "opening" of VAPs could have been caused by mechanical shearing during the purification process. The faces of VAPs in the open state were curved, most likely due to the characteristics of the opening process. Another characteristic feature of the open VAPs was their large size, with diameters exceeding 250 nm. This suggests that VAPs might need to reach certain dimensions before they can be opened.

The analysis of the VAP constituents revealed that the structure is composed of multiple subunits of a SIRV2-encoded protein, SIRV2-P98. No lipid component could be detected by the methods used. The absence or extremely low proportion of lipids agrees with the fact that VAPs could be isolated as intact structures after extensive washing of the membrane fraction with detergents (Materials and Methods).

SIRV2-P98 was confirmed to be the sole constituent of the VAP by heterologous expression experiments. Expression of SIRV2-ORF98 in both *S. acidocaldarius* and *E. coli* led to the formation of VAPs on cell membranes (Figs. 5 and 6). These results demonstrate that P98 is capable of self-assembling into pyramidal structures with seven-fold rotational symmetry. The involvement of certain auxiliary proteins in VAP assembly can be excluded. ORF98 was the only SIRV2 gene expressed in the mutant cells of *S*. *acidocaldarius* or *E. coli.* It is remarkable that the dramatic differences in the membrane composition and chemistry between bacterial and archaeal cells (7) did not affect the self-assembly of recombinant P98 into VAPs *in vivo.* The ability of P98 to form VAPs under the different conditions required for the growth of an hyperthermophilic, extremely acidophilic archaeon and a mesophilic bacterium is also remarkable.

The VAPs formed by heterologous expression of SIRV2-ORF98 in *E. coli and S*. *acidocaldarius* were similar in size and shape to those formed in *S*. *islandicus* infected with SIRV2. However, in the heterologous systems, VAPs were never observed in the opened state, unlike in the natural system where VAPs eventually open and cause perforation of the infected cell. These observations suggest that at least one special factor is required for the process of VAP opening, which is absent in *S. acidocaldarius* and *E. coli. ,* The nature and origin, viral or cellular, of this factor is currently unclear.

Our results demonstrate that the rudivirus SIRV2 encodes one autonomous structure in addition to the capsid, i.e., the VAP. The virus-encoded constituents of both structures can self-assemble. The major capsid protein self-assembles into filaments with the same diameter as the native linear virion (8), and VAP protein P98 self-assembles into pyramids. However, the functions of the two autonomous structures are different. Whereas the function of the former is DNA packaging and the formation of virus particles, the latter is specifically designed to release the virus particles from the host cell. To the best of our knowledge, the VAP represents the first example of a new class of non-capsid virus-encoded autonomous structures. The molecular simplicity and elegance of the VAP design revealed in this study should aid in the future analysis of the elaborate molecular mechanisms of the unique virion release system in Archaea.

### BIBLIOGRAPHY

1. Prangishvili, D., Forterre, P. & Garrett, R. A. (2006) Nat Rev Microbiol 4, 837-48.
2. Bize, A., Karlsson, E. A., Ekefjard, K., Quax, T. E., Pina, M., Prevost, M. C.,Forterre, P., Tenaillon, O., Bernander, R. & Prangishvili, D. (2009) Proc Natl Acad Sci USA 106, 11306-11.
3. Brumfield, S. K., Ortmann, A. C., Ruigrok, V., Suci, P., Douglas, T. & Young, M. J. (2009) J Virol 83, 5964-70.
4. Quax, T. E., Krupovic, M., Lucas, S., Forterre, P. & Prangishvili, D. Virology 404, 1-4.
5. Berkner, S., Wlodkowski, A., Albers, S. V. & Lipps, G. Extremophiles 14, 249-59.
6. Albers, S. V., Szabo, Z. & Driessen, A. J. (2003) J Bacteriol 185, 3918-25.
7. van de Vossenberg, J. L., Driessen, A. J. & Konings, W. N. (1998) Extremophiles 2, 163-70.
8. Vestergaard, G., Shah, S. A., Bize, A., Reitberger, W., Reuter, M., Phan, H.,Briegel, A., Rachel, R., Garrett, R. A. & Prangishvili, D. (2008) J Bacteriol 190, 6837-45.
9. Hohenberg H, Mannweiler K, Müller M (1994) High-pressure freezing of cell suspensions in cellulose capillary tubes. J Microsc 175:34-43
10. Albers SV, et al. (2006) Production of recombinant and tagged proteins in the hyperthermophilic archaeon Sulfolobus solfataricus. Appl Environ Microbiol. 72:102-111
11. Berkner S, Lipps G (2007) Characterization of the transcriptional activity of the cryptic plasmid pRN1 from Sulfolobus islandicus REN1H1 and regulation of its replication operon. J Bacteriol. 189:1711-21

## Claims

1. Homomultimeric protein structure constituted by assembled monomers of the P98 protein of *Sulfolobus islandicus* rod-shaped virus 2 (SIRV2) or assembled monomer variants of said P98 protein.

2. Homomultimeric protein structure according to claim 1, which consists in seven isosceles triangles, the area of which is constituted by assembled monomers of the P98 protein of *Sulfolobus islandicus* rod-shaped virus 2 (SIRV2) or assembled monomer variant of said P98 protein, wherein the base of each of these isosceles triangles forms the sides of a regular heptagon, in particular wherein the angle of the main vertex of said isosceles triangles is about 32°.

3. Homomultimeric protein structure according to claim 1 or 2, wherein the structure is a pyramid with 7 identical isosceles triangular faces and with a hollow base.

4. Homomultimeric protein structure according to claim 1 or 2, wherein the structure is in an open conformation.

5. Homomultimeric protein structure according to any one of claims 1 to 4, obtained by expression of the nucleic acid encoding the P98 protein of *Sulfolobus islandicus* rod-shaped virus 2 (SIRV2) or encoding a variant of the p98 protein in a cell, in particular in *Sulfolobus acidocaldarius* or in *Escherichia coli.*

6. Conjugate comprising or consisting of a homomultimeric protein structure as defined in any one of claims 1 to 5, to which one or more heterologous molecule(s) is covalently or non-covalently attached.

7. Lipid layer, in particular lipid bilayer, comprising inserted in the layer (or bilayer) or exposed on its surface, a homomultimeric protein structure or conjugate as defined in any one of claims 1 to 6.

8. Lipid layer or bilayer according to claim 7, which is a model lipid layer, such as black lipid membranes (BLM), supported lipid bilayers (SLB), the tethered bilayer lipid membrane (t-BLM) and vesicles.

9. Vesicle according to claim 8, which is an artificial vesicle, such as a micelle, bicelle, nanodisc or liposome.

10. Non-archae cell, expressing, in one of its membranes, the homomultimeric protein structure or conjugate as defined in any one of claims 1 to 6, in particular a prokaryotic cell, such as bacteria, for example *E*. *coli,* or an eukaryotic cell, such as a yeast, a plant cell, an animal cell, preferably a mammalian cell, more preferably a human cell.

11. Lipid layer, lipid bilayer, vesicle or non-archaea cell according to any one of claims 7 to 10, wherein, when said homomultimeric protein structure is a baseless 7-face pyramid, wherein the basis of the pyramid is in the lipid layer or bilayer and the apex of the pyramid is outside of the lipid layer or bilayer.

12. Lipid layer, lipid bilayer, vesicle or non-archaea cell according to claim 11, wherein the one or more heterologous molecule(s) is attached to the apex of the baseless 7-face pyramid, and is exposed outside of the lipid layer, of the lipid bilayer or of the cell membrane.

13. Use of a lipid layer or lipid bilayer according to any one of claims 7, 8, 11 or 12 to *in vitro* assay molecule-molecule interaction protein-protein interaction, in particular with the heterologous molecule(s) attached to the homomultimeric protein structure of the conjugate, or to analyze the genome, a transcriptome, a proteome or a metabolome.

14. Use of cell according to any one of claims 10 to 12, to *in vitro* study the cell membrane, the cell membrane dynamics or the interactions of cell membrane proteins.

15. Use of a micelle, bicelle, nanodisc, liposome or a non-archaea cell according to any one of claims 9 to 12 to transport and/or to expose *in vivo* the heterologous molecule(s) attached to the homomultimeric protein structure of the conjugate.
